(19) Europäisches Patentamt
European Patent Office
Office européen des brevets

(11) Publication number: 0 197 226 A1

(12) EUROPEAN PATENT APPLICATION

(21) Application number: 85400668.1

(22) Date of filing: 03.04.85

(51) Int. Cl.[4]: A 01 N 43/58
C 07 D 237/28

(43) Date of publication of application:
15.10.86 Bulletin 86/42

(84) Designated Contracting States:
AT BE CH DE FR GB IT LI LU NL SE

(71) Applicant: LAFARGE COPPEE
28, rue Emile Ménier
F-75782 Paris Cedex 16(FR)

(72) Inventor: Labovitz, Jeffrey Niles
185 Warley Street
Palo Alto California 94301(US)

(72) Inventor: Fang, Lawrence
225 Coronado Av,
Daly City CA. 94015(US)

(74) Representative: Phélip, Bruno et al,
c/o Cabinet Harlé & Phélip 21, rue de la Rochefoucauld
F-75009 Paris(FR)

(54) **Chemical hybridization agents comprising derivatives of cinnoline-3-carboxylic acids, and their application.**

(57) A chemical hybridization agent having the formula

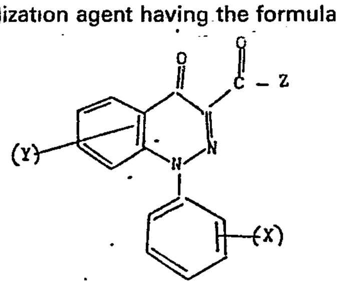

wherein X represents hydrogen or one to three groups, identical or different, selected from halogen, $CF_3$, $C_1$–$C_3$ alkyl, $C_1$–$C_3$ alkoxy or $C_1$–$C_4$ alkanoyl, Y represents hydrogen or one to three halogens, Z represents OR (wherein R represents H, a metal or ammonium cation, a $C_1$–$C_4$ alkyl group, a $C_1$–$C_4$ alkenyl group or a $C_1$–$C_4$ alkynyl group) or $NR_1R_2$ in which $R_1$ and $R_2$ are independently H, $C_1$–$C_4$ alkyl, $C_1$–$C_4$ alkenyl or $C_1$–$C_4$ alkynyl and agronomically acceptable salts thereof.

Method of sterilizing self-pollenizing plant, such as cereals, with said compounds and of producing hybrid seed of such plants.

EP 0 197 226 A1

# Chemical hybridization agents comprising derivatives of cinnoline-3-carboxylic acids, and their application

The present invention relates to a series of compositions containing cinnoline compounds and a method of producing hybrid cereal grain seed by application of such compositions.

Although genetic manipulation of plants through cross-breeding is a well-known process, hydrids of self-pollinating plants had been difficult to produce. In some cases, e.g., corn, intensive hand labor is required to prevent self-pollinating but is possible because the male and female flower parts are distant from each other on the corn stalk. However, in other plants, e.g., wheat, the male and female plant parts are contained within the same flower and self-pollination is difficult if not impossible to prevent. In wheat, the male stamen produces pollen inside a closed flower. The pollen then falls within the closed flower onto the female stigma. Only after this self-pollination step does the flower open to release extra pollen. Mechanical prevention of self-pollination as is practiced in corn is accordingly impossible in a plant such as wheat.

Nevertheless, it is possible to inhibit self-pollination in wheat and similar plants by chemically inhibiting the formation of pollen or by inducing the plant to produce non-functioning pollen. Several compounds have previously been developed which produce these effects.

US-A 4 345 934 discloses compounds of the formula:

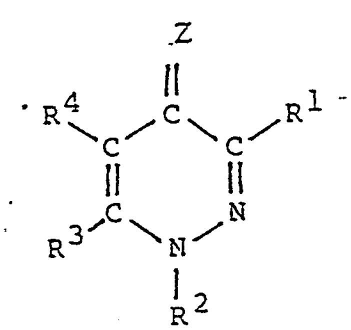

in which $R^1$ is carboxy, a carbamoyl group, or an alkoxy or aryloxy carbonyl group, $R^2$ is aryl or substituted aryl group, $R^3$ is alkyl or aryl, $R^4$ is hydrogen, alkyl, aralkyl or halogen and Z is oxygen or sulfur. These compounds are disclosed to be pollen suppressants, but the compound having formula

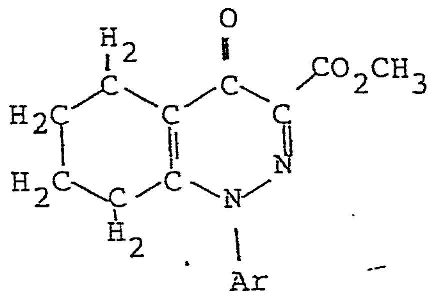

is set forth to be not active as a gametocide.

EP-A 0 037 133 discloses compounds of the formula:

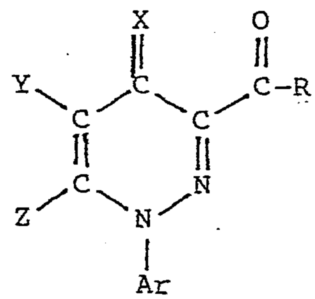

in which X represents oxygen or sulfur, Y represents hydrogen, halogen or an alkyl group, Z represents an alkyl group, Ar represents an optionally substituted phenyl group, and R represents a group which may be, among others $NR^1R^2$ or $ONR^1R^2$ in which $R^1$ can be hydrogen and $R^2$ can be an alkoxy group, an acyl group derived from a carboxylic or carbamic acid, or an alkyl group substituted with a carboxylic acid or ester group or may be $OR^3$ in which $R^3$ represents an alkyl or aryl group, eventually unsatured or substituted with various groups. These compounds are also disclosed to be pollen suppressants.

EP-A 0 049 971 discloses compounds of the formula:

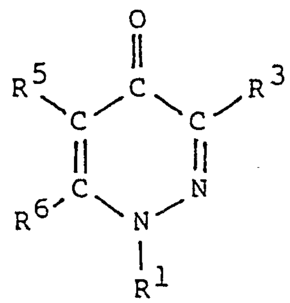

in which $R^1$ can be alkyl, phenyl, or naphthyl optionally substituted, $R^3$ can be H, carboxy or an alkali metal salt thereof, an alkoxy carbonyl, or a substituted carbamoyl, $R^5$ is a carboxy derivative of the type defined for $R^3$, and $R^6$ is a $C_1$-$C_4$ alkyl group of phenyl or naphtyl optionally substituted. These compounds are disclosed to be chemical hybridizing agents which operate by causing male sterility.

Synthesis (1) 52-3 (1983), as abstracted in chem. Abs. 98 143356e, discloses compounds of formula

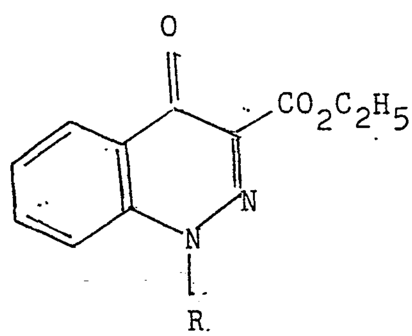

wherein R is phenyl or substituted phenyl. However, this publication is directed only to chemical synthesis and no use for the compound is disclosed.

Nevertheless, many of the compounds so tested have adverse effects on hybrid seed quality or injure plants at doses only slightly above those required to produce maximum male plant sterility. Accordingly, a continued need for new pollen

suppressants useful for producing hybrid seed of cereal grain exists.

Accordingly, it is an object of this invention to provide chemical hybridization agents for producing hybrid seed of cereal grain plants.

It is a further object of this invention to provide a method of suppressing pollen production in cereal grain plants using the cinnoline-3-carboxylic acid derivatives.

It is still a further object of this invention to provide a method for producing hybrid seed of cereal grain plants using the novel chemical sterilants of the invention.

These and other objects of the invention, as will hereinafter become more readily apparent, have been accomplished by providing a chemical pollen suppressant of the formula I

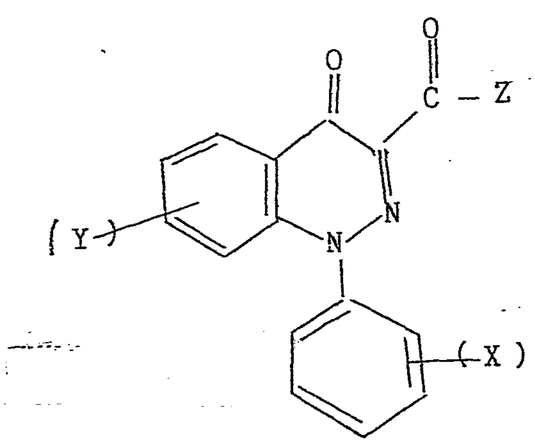

I

wherein X represents hydrogen or one to three groups, identical or different, selected from halogen, $CF_3$, $C_1$-$C_3$ alkyl, $C_1$-$C_3$ alkoxy or $C_1$-$C_4$ alkanoyl,Y represents hydrogen or one to three halogens, Z represents OR (wherein R represents H, a metal or ammonium cation, a $C_1$-$C_4$ alkyl group, a $C_1$-$C_4$ alkenyl group or a $C_1C_4$ alkynyl group) or $NR_1R_2$ in which $R_1$ and $R_2$ are independently H, $C_1$-$C_4$ alkyl,

$C_1$-$C_4$ alkenyl or $C_1$-$C_4$ alkynyl when Z is OR, and R a cation, R can be an alkali metal ion, as sodium or potassium, an alkaline earth metal ion, as magnesium, calcium and barium or a transition metal ions, as zinc, manganese, iron, or $NH_4^+$ or ($C_1$-$C_4$) alkyl substituted ammonium ion.

Also included within the scope of the invention are agronomically acceptable acid and base salts of compounds having the general formula I given. Typical acid addition salts are those formed with strong acids such as hydrochloric, hydrobromic, sulfuric and nitric acids by the protonation of a ring or side chain nitrogen. Salts of acidic functional-group substituents are also included in this invention. Throughout this application, agronomically acceptable salt means that the salt is not substantially more toxic to the plant or to the consumer of the plant than the parent compound from which the salt is formed.

Preferred compounds are those in which, Y is one or two halogens and more preferably Y is 5-F, and in which X
In one preferred embodiment COY is a carboxy group or a salt thereof.

The compounds of the invention can be synthesized according to known methods for the production of analogous compounds or can be produced by synthetic modification of known oxo-cinnolines. For example, numerous synthetic pathways to cinnolines are disclosed in Condensed Pyridazines Including Cinnolines and Phthalazines, R. N. Castle, ed., John Wiley and Sons, N.Y., 1973, pages 1-321 (cinnolines) and 761-1056 (pyridazines condensed with heterocyclic rings) of which are herein incorporated by reference. For example, one suitable method

0197226

involves the hydrolysis of readily accessible diethyl mesoxalate diphenylhydrazones of the formula:

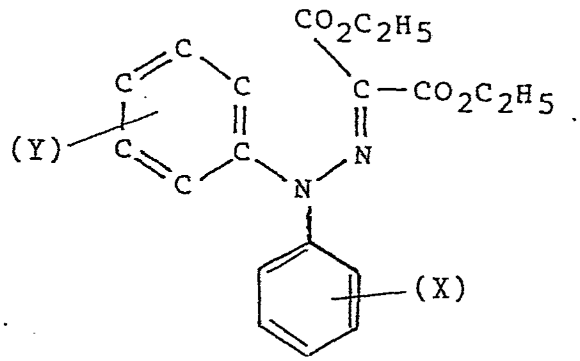

in which X and Y represent one of the groups previously named, and at least one orthoposition of the diphenyl hydrazone is free, to give a dicarboxylic acid. This acid is converted into a diacid chloride using a suitable reagent, such as thionyl chloride. The acid chloride then undergoes a Friedel-Crafts acylation reaction, for example in nitrobenzene at about 100°C in the presence of $TiCl_4$. A product of formula I wherein Z is OH. Groups that would interfere with the ring-forming reaction may be present in protected form (*e.g.*, an acylamino group that may later be converted into an amine) or they may be added later (*e.g.*, by halogenation of the phenyl rings) or they may be prepared by conversion of a suitable group present during synthesis (*e.g.*, the above-mentioned amino group may be diazotized and converted into many different functional groups).

Another general synthetic method for synthesizing compounds of the invention is described in *Synthesis*, previously cited. In this reaction sequence, the key step is condensation of an inter-

mediate of the formula:

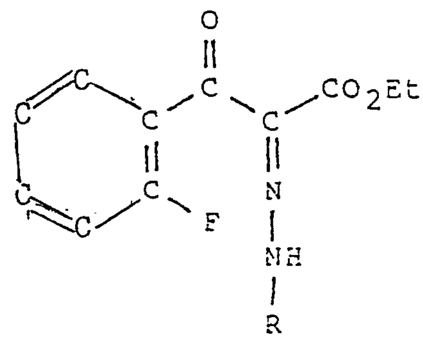

where R is an aryl or alkyl group and F may optionally be a nitro group rather than fluorine. This reaction is particularly useful for the preparation of cinnolines.

The above-indicated 3-carboxycinnolines can then be converted into other compounds of the invention by known methods. For example, the carboxylic acid group can be converted into a carboxylate salt or into an ester, or a protected amino group can be deprotected, diazotized, and converted into a different functional group.

Various modifications of these reactions and of other reactions capable of modifying the initially formed cyclic compounds can be used to produce all the compounds of the present invention, for example as is disclosed in the prior art patents previously cited (U.S. 4,345,934, EP-A 37133, and EP-A 49971), which are herein incorporated by reference.

Compounds of the invention are useful as chemical hybridization agents in gramineous or vegetable crops, such as wheat, barley, maize, rice, sorghum, millet, oats, rye, triticale, forage crops

and the like. Of these, treatment of wheat is preferred. Different plant growth regulating effects will be obtained depending upon the growth stage of the plant when treated. Compounds of the invention induce selected male sterility without also inducing unacceptable female sterility. About 30% female fertility is generally acceptable, although this level may differ when the method is used commercially, based on the economics of $F_1$ seed production. As used herein, the term male sterility includes sterility caused by lack of male flower parts, by formation of sterile pollen, and by male flower parts which produce normal pollen but are functionally unable to cause pollination. Where the male sterility of compounds of the invention is accompanied by female infertility of an unacceptable level or by phytotoxicity, compounds are still minimally useful in production of ergot, for example as described in FR-A-2 400 832 which is herein incorporated by reference.

When compounds of the invention are used in hybridization, they are used in an amount sufficient to produce the effect of male sterility without producing a phytotoxic reaction or other undesired side-reaction. Compounds of the invention are generally applied at a rate of from 0.025 to 20.0 kg per hectare and preferably from 0.125 to 10.0 kg per hectare. The amount used depends upon the plant type and the method of application as is well-known to those skilled in the art and can be determined by simple previous experimentation if not known.

Although any method of hybridization may be used, the following method generally is sufficient. The two parent strains to be crossed

are planted in alternate sections, rows, or groups of rows. The female parent is treated with a compound of the invention in order to render this parent male sterile. Pollen from the male (untreated) parent then fertilizes the female parent, either by means of human intervention or preferably by means of a natural process, such as wind-borne pollination. The seed produced by the female parent is an F-1 hybrid, which is then collected according to usual techniques.

One method of applying the compounds of the invention in the previously-mentioned hybridization technique or for otherwise inducing male sterility is application directly to the plant leaves. When this method is used, very selective male sterility can be obtained when the compound is applied between the beginning of bloom and the beginning of meiosis.

Compounds of the invention can also be applied directly to seed in order to cause male sterility, whereby the seeds are dipped into a fluid formulation containing the active ingredient. Seed can also be sprayed with a solution or suspension containing a compound of the invention. In general, seed are treated with a compound of the invention in an amount of from about 1/4 to 10 kg per 100 kg of seed.

Compounds of the invention are also effective when they are applied to the medium in which plants are grown such as soil or the water surface in a rice field.

Compounds of the invention can be used as hybridization materials together with other plant regulatory agents, for example, in mixtures with these compounds. Examples of plant regulating materials which can be used include auxins, gibberellins, ethylene liberating materials such as Ethephon, pyridones, cytokinins, maleic hydrazide, carbonic acid, 2,2-dimethyl hydrazide, cholines (as well as their salts), (2-chloroethyl)trimethylammonium chloride, triiodobenzoic acid, tributyl-2,4-dichlorobenzene-phosphonium chloride, polymeric N-vinyl-2-oxazolidinones, tri(-dimethylaminoethyl)phosphate, and salts of these compounds as well as N-dimethylamino-1,2,3,6-tetrahydrophthalamides and their salts. Compositions containing one or more compounds of the invention in a 1:99-99:1 ratio to one or more different compounds having plant regulatory activities may be prepared. Likewise, compounds of the invention may be prepared into compositions useful for other agricultural purposes, such as herbicides, fungicides, insecticides and plant bactericides.

A compound of the invention can be applied to a plant either as itself or in combination with other plant growth regulators. A composition containing a compound of the invention and any other active ingredient may be diluted with an agronomically suitable carrier, which is any substance which itself is without any significant effect on plants but which is added in order to allow simpler application of the active ingredients to plants. Carriers include both liquids and solids. Accordingly, compositions of the invention can be either solid or liquid formulations or solutions. For example, the compounds can be used in powders, emulsifiable concentrates, dusts, pellets, aerosols and solutions. In any of the various formulations, a surface active agent may be added in order to increase uptake of the active compounds. It

is especially preferred, and particular for methods which involve application to leaves, to utilize agents which aid in the application of the material, for example, dispersion agents and detergents.

Compounds of the invention can be dissolved in any suitable solvent. Examples of solvents which can be used include water, alcohols, ketones, aromatic hydrocarbons, halogenated hydrocarbons, dimethylformamide, dioxane, and dimethylsulfoxide. Mixtures of these solvents can likewise be used. The concentration of these solutions can be from about 2 to about 98% by weight of active ingredient and is preferred to be in the range from about 20 to about 75% by weight.

In order to produce emulsifiable concentrates, the compounds of the invention are dissolved in an organic solvent, such as benzene, toluene, xylene, methylated naphthalene, corn oil, terpentine, o-dichlorobenzene, isophorone, cyclohexane, or methyl oleate or in mixtures of these solvents, together with an emulsifying material which allows the dispersion in water. Suitable emulsifying agents include ethylene oxide derivatives of alkylphenols or long-chained alcohols, mercaptans, carboxylic acids, and reactive amines, and especially high molecular weight alcohols. Solvent-soluble sulfates or sulfonates, such as the alkaline earth salts or amine salts of alkylbenzenesulfonates as well as sodium fatty alcohol sulfates with surface active properties can be utilized as emulsifying agents either alone or in combination with an ethylene oxide reaction product. Free-flowing emulsion concentrates are formulated similarly to emulsifiable concentrates and contain, in addition to the previously described components,

water as well as a stabilizing agent, such as a water-soluble cellulose derivative or a water-soluble salt of a polyacrylic acid. The concentration of the active ingredient in the emulsifiable concentrate is generally about 10 to 60 wt. % and in free-flowing emulsion concentrates is generally about 10 to 60% or sometimes up to 75% by weight.

When a powder containing the compound of the invention is being prepared, the active ingredient is usually mixed with a finely divided solid, such as a clay, an organic silicate or carbonate, or a silica gel along with an agent capable of holding together the resulting materials. The concentration of the active ingredient in such powders generally lies between about 20 and 98% by weight and preferably lies between 40 and 75% by weight. A dispersion material can generally be present in an amount of about 0.5 to 3% by weight of the entire powder. An agent may be added in order to control water absorption and if added is generally present in an amount of about 0.1 to about 5% by weight of the total powder.

Dusts can be prepared by mixing the active ingredient with a finely divided inert solid, which can be of an organic or inorganic nature. Suitable material for this purpose include flour, farina, diatomite, silicates, carbonates, and clays. A satisfactory method for the production of dusts involves crushing a wettable powder together with a finely divided carrier. A dust concentrate, which contains from about 20 to about 80% of the active ingredient, is produced according to known methods and then diluted to form a final concentration of the compound of the invention of about 1 to about

10% by weight of the dust.

Particulate formulations can be prepared by any known method, for example by impregnating the active ingredient into a solid material, such as particulate Fullers earth, vermiculite, cornmeal, seed hulls such as grain hulls, or other materials. A solution of one or more of the compounds of the invention in a freely flowing organic solvent can be applied to the particulate solid or mixed therewith, after which the solvent is evaporated away. The particulate material is not limited to a particular size. However, a useful size is from 16 to 60 mesh (U.S. standard mesh size). The active ingredient generally occupies about 2 to about 15 wt % of the particulate formulation.

Salts of the compounds of the invention can be prepared as aqueous solutions or suspensions and applied in this form. The salts occupy typically about 0.05 to about 50 wt. % and preferably from about 0.1 to 10 wt. % of the composition. In any event, these compositions may be diluted with additional water prior to use. In some cases the activity of the active material can be increased by including another agent in the composition, such as glycerin, methylethylcellulose, hydroxyethyl cellulose, polyoxyethylene sorbitol mono-oleate, polypropylene glycol, polyacrylic acid, polyethylene sodium malonate, or polyethyleneoxide. The auxiliary occupies generally from about 0.1 to about 5 wt. % and particularly from about 0.5 to 2 wt. % of the resulting composition. The various compositions can in any case also contain an agriculturally suitable surface active agent.

The compounds of the invention can be

applied according to any known methods, for example in the form of hydraulic sprays, air sprays or dusts. For methods which involve the application of small volumes, a solution of the compound is generally utilized. The volume used and the rate of application depend upon various factors which vary with the method used, such as the specific type of application method, the stage of development of the plant to which the active ingredient is being applied, and other factors well known to those skilled in the art or easily determined by simple experimentation.

Having now generally described this invention, the same will be better understood by reference to certain specific examples which are included herein for purposes of illustration only and are not intended to be limiting of the invention or any embodiment thereof, unless so specified.

Example 1: Synthesis of potassium 1-(4'-trifluoromethylphenyl)-5-fluoro-1,4-dihydro-4-oxo-cinnoline-3-carboxylate

a) to 3,6 g (25 mmole) of 2,2-dimethyl-1,3-dioxane-4,6 dione (Meldrum's acid) and 6,1 g of 4-dimethylamino-pyridine in 60 ml of methylenechloride was added with stirring, 4,4 g (25 mmole) of 2,6-difluorobenzoylchloride. The temperature was maintained at 0°C throughout the addition period and for three additional hours of stirring, under argon atmosphere. Most of the methylene chloride was then removed by distillation at 25°C or less using a water aspirator to maintain reduced pressure. There was then added 60 ml of anhydrous methanol containing 25 mmole of concentrated sulfuric acid. The mixture was refluxed for three hours and after concentration in vacuo the residue was taken up in

methylene chloride and washed with water. The organic layer was dried over anhydrous $Na_2SO_4$, the solvent was removed in vacuo and the residual oil was distilled to give 4.5 g of methyl 2,6-difluorobenzoylacetate.

b) To 290 g of 4-aminobenzotrifluoride in 1000 ml of methanol was added 540 ml of conc. hydrochloric acid with ice water cooling. There was then added at 0-5°C, 124.4g of $NaNO_2$ in 400 ml of $H_2O$, while stirring was vigorously maintained. The resulting solution of diazonium salt was then added, with stirring at 5-10°C, to a solution of methyl 2,6-difluorobenzoyl-acetate (321g) in 2000 ml of methanol containing 441g of potassium acetate. The resulting precipitate was collected by filtration, dissolved in methylene chloride and dried over anhydrous sodium sulfate. Solvent was removed in vacuo and the residual solid was dried further under vacuum overnight at room temperature. The crude adduct (300g) was then dissolved in 200 ml of dry dimethylformamide. To this solution was added 118g of anhydrous $K_2CO_3$ and 100 mg of 18-crown-6 (crown-ether available from Aldrich Chem. Co.). The reaction mixture was then heated with continual stirring to 110°-120° for 1.5 hr, under an argon atmosphere. Approximately three-fourths of the solvent was then removed in vacuo at 90°C. The residue was poured into water with stirring. There was obtained 280g of solid ester after filtration and drying. (mp 196-198°C). Hydrolysis of 280g of the ester obtained above in 3000 ml of methanol containing 64g of potassium hydroxide, was carried out at room temperature. The resulting potassium salt was obtained by concentration and filtration followed by washing of the solid salt with acetone.

0197226

A sample of the free acid melted at 241-243°C.

Example 2 to 36:

Table I lists typical compounds of the invention, having formula II, with their melting points, prepared by conventional methods.

Table I

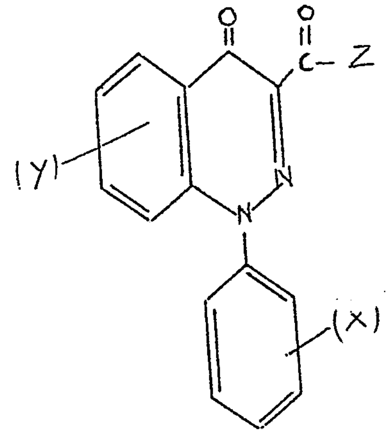

| Example n° | X | Y | Z | mp(°C) |
|---|---|---|---|---|
| 2 | H | 5-F | $OCH_2CH_3$ | |
| 3 | H | 5F | OH | 247-249°C |
| 4 | H | 5-Cl | OH | |
| 5 | H | 5-Cl | $OCH_2CH_3$ | 128-130°C |
| 6 | 2'-F | 5-F | OH | 220-222°C |
| 7 | 2'-F | 5-F | $OCH_3$ | |
| 8 | 3'-F | 5-F | OH | 221-223°C |
| 9 | 3'-F | 5-F | $OCH_2CH_3$ | 185-187°C |
| 10 | 4'-F | 5-F | OH | 219°C |
| 11 | 4'-F | 5-F | $OCH_2CH_3$ | 168-170°C |
| 12 | 3'-Cl | 5-F | OH | |
| 13 | 3'-Cl | 5-F | $OCH_2CH_3$ | 189-190°C |
| 14 | 4'-Cl | 5-H | OH | |
| 15 | 4'-Cl | 5-Cl | OH | |
| 16 | 4'-Cl | 5-Cl | $OCH_2CH_3$ | 148-150°C |
| 17 | 4'-Cl | 5-F | $NH_2$ | 224-227°C |
| 18 | 4'-Cl | 5-F | $NHCH_3$ | 217-219°C |
| 19 | 4'-Cl | 5-F | $NHC_2H_5$ | 195-197°C |
| 20 | 4'-Cl | 5-F | $N(C_2H_5)_2$ | 223-225°C |
| 21 | 4'-Cl | 5-F | $NH-CH_2CH=CH_2$ | 228-230°C |
| 22 | 4'-Cl | 5-F | $NHCH_2COOCH_3$ | |
| 23 | 4'-Br | 5-F | OH | 251-253°C |
| 24 | 4'-Br | 5-F | $OCH_2CH_3$ | 195-197°C |

| | | | | |
|---|---|---|---|---|
| 25 | 4'-I | 5-F | OH | |
| 26 | 4'-I | 5-F | $OCH_2CH_3$ | 179-181°C |
| 27 | 3'-$CF_3$ | 5-F | OH | 259-261°C |
| 28 | 3'-$CF_3$ | 5-F | $OCH_3$ | 196-198°C |
| 29 | 4'-$CF_3$ | 5-F | $OCH_2CH_3$ | 196-198°C |
| 30 | 4'-$CF_3$ | 5-F | $NHCH_2CH_3$ | 227-229°C |
| 31 | 4'-$CF_3$ | 5-F | $N(CH_2CH_3)_2$ | 228-230°C |
| 32 | 4'-$CH_3$ | 5-F | OH | |
| 33 | 4'-$CH_3$ | 5-F | $OCH_2CH_3$ | 124-127°C |
| 34 | 4'-$OCH_3$ | 5-F | OH | |
| 35 | 4'-$OCH_3$ | 5-F | $OCH_2CH_3$ | 151-154°C |
| 36 | 4'-$COCH_3$ | 5-F | $OCH_3$ | 185°C |
| 37 | 3',4'-diCl | 5-F | OH | 188-190°C |
| 38 | 3'-$CF_3$,4'Cl | 5-F | $OCH_3$ | 185-188°C |

Studies of biological activities

A biological assay for pollen suppression was conducted on different wheat varieties, i.e. Marris Hobbit, Stephens, Hill 81 and W-41 (Anza); W-41 is a heavy tillering wheat which is grown commercially in California.

Seeds were planted to provide four plants per 8-inch pot. Plants were raised in a greenhouse until the stage indicated in the following table of results. These different stages of growth were defined for the purposes of this experiment as follows: Stage 1, spike length of 0.1-0.5 cm; Stage 2, spike length of 0.5-1.5 cm; Stage 3, spike length of 1.5-2.5 cm. External appearance was correlated with the development of the spikelet in order to avoid mistaking the onset of meiosis. Spikelets were removed at various developmental stages and anthers were removed from the most mature florets (which generally occured in about the middle of the spiklet). The anthers were crushed in acetocarmine or propeocarmine and the

state of pollen development was assessed. Cytological examinations were made to assess the best time for application. Compounds were applied on the leaves as solutions in water or water/acetone (5-50% acetone) or as aqueous emulsions. In all cases, 0.1% Triton X-100 was used as a wetting agent. Plants were sprayed to runoff with a test solution and then replaced in such a way that control plants were interspersed with treated plants. Heads were bagged upon emergence and seed set was used as a measure of sterility induction. Compounds that demonstrated good sterilization ability were tested for their effect on female fertility by cross-pollination of awned female plants with awnless male pollen donors.

Other tests were performed in fields in order to evaluate the activity of the compounds for inducing male sterility of wheat. The compounds were applied in water with suitable surfactants and emulsifiers where necessary. Delivery volumes range from 100 to 1000 liters per hectare and application rates range from 0.05 to 10 kg per hectare. Treated plants are surrounded by an untreated pollen donor and degree of sterilization is monitored by bagging heads of treated plants and counting the number of seeds at maturity. Degree of outcrossing is determined by counting seeds in unbagged heads. A male dominant marker, such as a colored aleurone, in the progeny seed is used to confirm true outcrossing. In some cases the application rate can change continuously at a known rate along the length of the treated plot.

Control studies were conducted using a known prior art compound (1-(4-chlorophenyl)-1,4-dihydro-4-oxo-6-methylpyridazine-3-carboxylic acid).

Optimal dosage and correct stage of application of this compound were determined in order that test crosses could be compared to test crosses made using the compounds of the invention.

The results obtained in green house (Table II) and in fields (Table III) using the general procedures described above, are mentioned in the following.

Table II

| Example n° | Stage | Dose (ppm) | % male sterility |
|---|---|---|---|
| 1 (ester $CH_3$) | 3 | 60 | 100 |
| 2 | 2 | 25 | 90 |
| 4 | 2 | 50 | 100 |
| 6 | 3 | 50 | 100 |
| 8 | 2 | 100 | 98 |
| 9 | 2 | 100 | 85 |
| 10 | 1 | 25 | 100 |
| 12 | 2 | 100 | 50 |
| 14 | 1 | 250 | 80 |
| | | 125 | 26 |
| 15 | 1 | 80 | 80 |
| 16 | 1 | 40 | 40 |
| 25 | 1 | 80 | 100 |
| 26 | 1 | 80 | 98 |
| 27 | 2 | 250 | 92 |
| 32 | 1 | 60 | 30 |
| 33 | 1 | 40 | 25 |
| 34 | 1 | 60 | 32 |
| 35 | 1 | 60 | 30 |
| 37 | 2 | 25 | 85 |
| | | 125 | 98 |
| Control | - | 62.5 | 96 |
| | | 31,25 | 82 |

Table III

| Wheat variety | Example n° | Rate | % male sterility | % female fertility |
|---|---|---|---|---|
| Marris Hobbit | 1 | 0.5 kg/ha | 98 | 48.5 |
| | 4 (potassium salt) | 0.4 kg/ha | 98 | 54 |
| Stephens | 1 | 0.5 kg/ha | 99 | 44 |
| | 4 (potassium salt) | 0.4 kg/ha | 99 | 64 |
| Hill 81 | 1 | 0.5 kg/ha | 99 | 54 |
| | 4 (potassium salt) | 0.4 kg/ha | 98 | 71 |

The invention now being fully described, it will be apparent to one of ordinary skill in the art that many changes and modifications can be made thereto without departing from the spirit or scope of the invention as set forth herein.

CLAIMS

1. A chemical hybridization agent having the formula

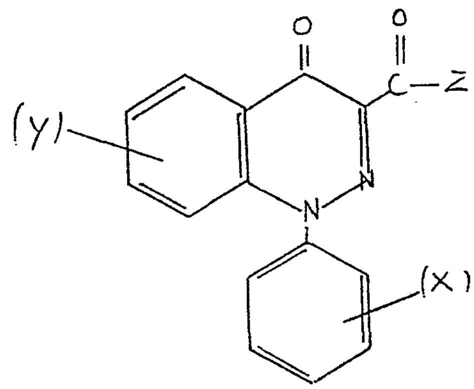

wherein X represents hydrogen or one to three groups, identical or different, selected from halogen, $CF_3$, $C_1$-$C_3$ alkyl, $C_1$-$C_3$ alkoxy or $C_1$-$C_4$ alkanoyl, Y represents hydrogen or one to three halogens, Z represents OR (wherein R represents H, a metal or ammonium cation, a $C_1$-$C_4$ alkyl group, a $C_1$-$C_4$ alkenyl group or a $C_1$-$C_4$ alkynyl group) or $NR_1R_2$ in which $R_1$ and $R_2$ are independently H, $C_1$-$C_4$ alkyl, $C_1$ -$C_4$ alkenyl or $C_1$-$C_4$ alkynyl and agronomically acceptable salts thereof.

2. A chemical hybridization agent of claim 1 which is 1-(4-trifluoromethylphenyl)-1,4-dihydro-4-oxo-5-fluorocinnoline-3-carboxylic acid or an agronomically acceptable salt thereof.

3. A method of inducing male sterility in a plant which comprises treating the plant, a seed from which said plant is to be grown, or a medium in which said plant is growing or is to be grown with a compound of any claim 1 to 2.

4. A method of producing hybrid seed from a self-pollenizing plant which comprises

sterilizing the male anthers of a female parent plant by treating the plant with an effective amount of at least one compound of any claim 1 to 2, and pollinating said female parent with pollen from an untreated male parent, thereby allowing production of said hybrid seed.

5. A method of claim 3 or 4, wherein said plant is a cereal plant.

6. A method of claim 5, wherein said cereal plant is wheat.

European Patent Office

# EUROPEAN SEARCH REPORT

0197226

Application number

EP 85 40 0668

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | EP-A-0 138 661 (LAFARGE COPPEE) * Claims * | 1-6 | A 01 N 43/58<br>C 07 D 237/28 |
| A,D | US-A-4 345 934 (T.T. FUJIMOTO) * Abstract * | 1-6 | |
| A,D | EP-A-0 037 133 (SHELL) * Claims * | 1-6 | |

TECHNICAL FIELDS SEARCHED (Int. Cl.4)

A 01 N
C 07 D

The present search report has been drawn up for all claims

| Place of search | Date of completion of the search | Examiner |
|---|---|---|
| THE HAGUE | 06-12-1985 | DECORTE D. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document